# EUROPÄISCHE PATENTSCHRIFT

(11) **EP 2 178 480 B1**
(45) Veröffentlichungstag und Bekanntmachung des Hinweises auf die Patenterteilung: **27.10.2010**
(21) Anmeldenummer: 08785540.9
(22) Anmeldetag: 14.08.2008
(51) Int. Cl.: A61F 13/02

(54) **VERFAHREN ZUM MEHRSPURIGEN KONFEKTIONIEREN TRANSDERMALER THERAPEUTISCHER PFLASTER**
METHOD FOR THE MULTI-TRACK TAILORING OF TRANSDERMAL THERAPEUTIC PATCHES
PROCÉDÉ POUR CONFECTIONNER PLUSIEURS BANDES DE PANSEMENTS TRANSDERMIQUES

(30) Priorität: 21.08.2007 US 965616 P
(43) Veröffentlichungstag der Anmeldung: 28.04.2010
(73) Patentinhaber: LTS LOHMANN Therapie-Systeme AG, 56626 Andernach (DE)
(72) Erfinder: HILLE, Thomas, 56073 Koblenz (DE); STEINBORN, Peter, 56567 Neuwied (DE)
(74) Vertreter: Schmidt, Werner
(86) Internationale Anmeldenummer: PCT/EP2008/006678
(87) Internationale Veröffentlichungsnummer: WO 2009/024284

(56) Entgegenhaltungen:
- EP-A- 1 438 943
- DE-A1- 4 110 027
- DE-A1- 19 738 855
- DE-C1- 4 232 279
- DE-C1- 4 406 976
- DE-C1- 19 547 691

## Beschreibung

Die Erfindung betrifft ein Verfahren zum mehrspurigen Konfektionieren transdermaler therapeutischer Pflaster mit einer mindestens einlagigen, wirkstoffhaltigen und haftklebenden Reservoirfolie, die zwischen einer Rückfolie und einer ablösbaren Schutzfolie angeordnet wird.

Transdermale therapeutische Pflaster sind auf die Haut aufzubringende Arzneiformen mit dem Aussehen traditioneller Pflaster. Die Pflaster enthalten mindestens einen über die Haut abzugebenden Arzneistoff. Die Arzneistoffe werden in vorbestimmter Rate kontinuierlich über einen festgelegten Zeitraum an einem festgelegten Anwendungsort auf der Körperhaut abgegeben.

Derartige Pflaster haben in der Regel einfache geometrische Formen. Sie sind in der Draufsicht gesehen z.B. rechteckig oder rautenförmig. Das Pflaster selbst besteht z.B. aus einem wirkstoffhaltigen Folienreservoir mit einer zur Haut hin orientierten Klebeschicht. Auch die Klebeschicht kann zusätzlich Arzneistoff tragen. Das Folienreservoir ist verpackt zwischen einer ablösbaren Schutzfolie und einer zur Schutzfolie hin klebenden Rückfolie. Letztere verhindert, dass Dritte bei einem unbeabsichtigten Pflasterkontakt Wirkstoffe aufnehmen können.

Derartige Pflaster werden bisher nach einem aus der DE 41 10 027 C2 bekannten Verfahren zur einspurigen Konfektionierung hergestellt. Bei diesem Verfahren liegt die Reservoirfolie als lange Bahn vor, die zu einer schmalen Rolle aufgewickelt wird. Der Durchmesser dieser Rolle ist in der Regel ein vielfaches größer als die Rollenbreite. Soll nun zur Steigerung der Fertigungsmenge pro Zeiteinheit auf einer Verpackungsmaschine mehrere dieser schmalen Rollen nebeneinander zur mehrspurigen Konfektionierung verwendet werden, müssen die einseitig klebrigen bahnförmigen Reservoirfolien synchron an die Schutzfolie herangeführt und auf Länge geschnitten der Verpackungsvorrichtung übergeben werden. Da die Folienrollen meist unterschiedliche Durchmesser haben, werden die bahnförmigen Reservoirfolien unterschiedlichen Zugspannungen ausgesetzt, was die Synchronisation negativ beeinflusst. Das synchrone Heranführen ist jedoch zwingend erforderlich, um beim mehrnutzigen Schneiden der bahnförmigen Reservoirfolien keinen Abfall entstehen zu lassen. Wirkstoffhaltige Abfälle stellen einen aufwendig zu entsorgenden Sondermüll dar.

Der vorliegenden Erfindung liegt daher die Problemstellung zugrunde, ein Verfahren zum mehrspurigen Konfektionieren zu entwickeln, bei dem von Rollen abwickelbare bahnförmige Reservoirfolien verwendet werden können, ohne wirkstoffhaltige Abfälle zu erzeugen.

Diese Problemstellung wird mit den Merkmalen des Hauptanspruches gelöst. Hierbei liegt pro Konfektionierungsspur die Reservoirfolie jeweils als Reservoirfolienbahn auf einer Rolle aufgewickelt vor. Die Reservoirfolienbahnen werden pro Spur mit ihrer Haftklebeschicht auf ein diskontinuierlich in Längsrichtung der Reservoirfolienbahnen bewegtes Transportelement aufgelegt. Auf dem Transportelement werden die nebeneinander aufliegenden Reservoirfolienbahnen quer oder schräg zur Längsrichtung zumindest annähernd zeitgleich in einzelnen Reservoirfolien mit Hilfe einer Trennvorrichtung abgetrennt. Anschließend werden die jeweils paarweise nebeneinander liegenden Reservoirfolien am Transportelementende an ein kontinuierlich bewegtes weiteres Transportelement zur beabstandenden Ablage übergeben. Nach der Übergabe der vorderen Reservoirfolien wird das abgebende Transportelement kurz abgebremst.

Weitere Einzelheiten der Erfindung ergeben sich aus den Unteransprüchen und der nachfolgenden Beschreibung eines schematisch dargestellten Ausführungsbeispiels.
- Figur 1:: Synchronisationsstation für z.B. zwei schmale Rollen;
- Figur 2:: Synchronisationsstation mit Einmaltransportfolie sowie Schneidevorrichtung und Verpackungsstation;
- Figur 3:: wie Figur 2, jedoch Synchronisationsstation mit einem Endlostransportband;
- Figur 4:: Draufsicht auf ein fertig verpacktes Pflaster;
- Figur 5:: Querschnitt durch aneinander gereihte, noch ungetrennte Pflaster.

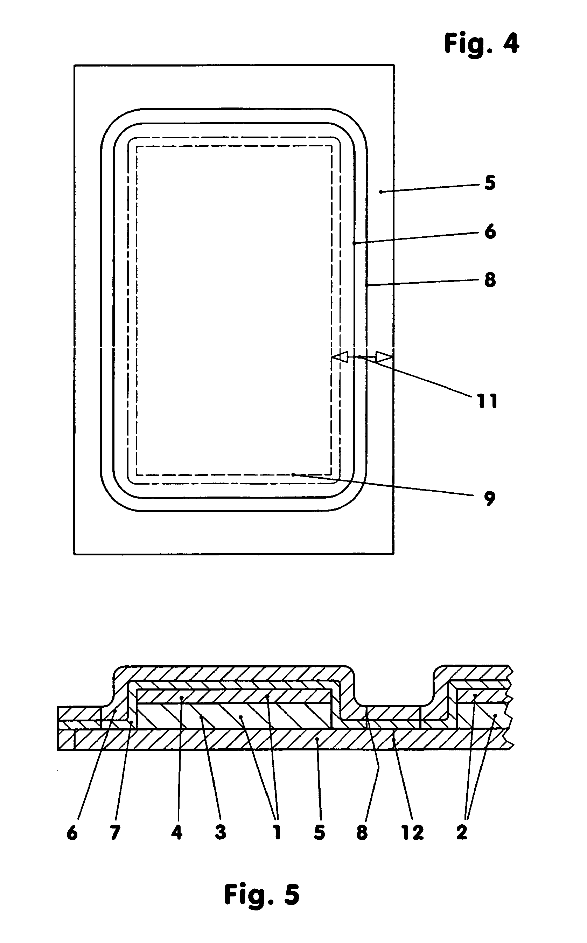

Die Figuren 4 und 5 zeigen ein transdermales therapeutisches Pflaster in der Draufsicht und im Querschnitt. Die in den Figuren 4 und 5 dargestellten Folien- und Schichtdicken sind überhöht, also im Verhältnis zur Pflasterlänge und -breite nicht maßstäblich.

Das Pflaster umfasst im Wesentlichen eine wirkstoffhaltige Reservoirfolie (1, 2), eine Schutzfolie (5) und eine Rückfolie (6). Die einzelne hier rechteckige Reservoirfolie (1,2) besteht beispielsweise aus der wirkstoffhaltigen Haftklebeschicht (3) und einer Sperrschicht (4). Letztere verhindert u.a. die rückseitige Wegdiffusion des Wirkstoffs. Das Laminat aus Haftklebeschicht (3) und Sperrschicht (4) liegt mittig und klebend auf der Schutzfolie (5) auf. Hierbei steht die Schutzfolie (5) an allen Rändern über die Reservoirfolie (1, 2) über.

Über der Reservoirfolie (1, 2) liegt die mit einer Klebeschicht (7) ausgestattete Rückfolie (6). Die Klebeschicht (7) kontaktiert hierbei die Sperrschicht (4) und die Randbereiche der Schutzfolie (5). Die Schutzfolie (5) und die Rückfolie (6) umschließen die Reservoirfolie (1, 2) wirkstoffdicht, wobei die Schutzfolie (5) im Ausführungsbeispiel über die Rückfolie (6) übersteht.

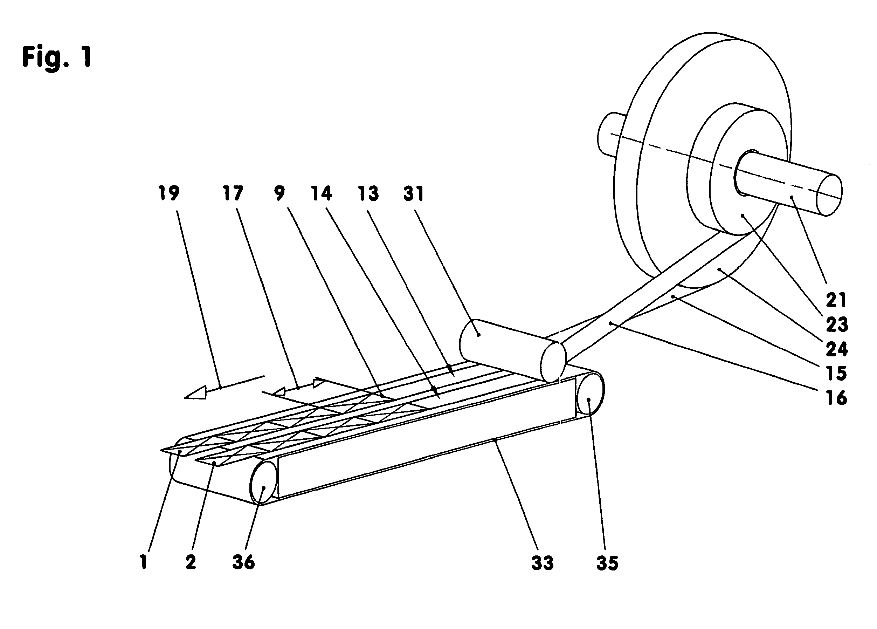

Um derartige Pflaster kostengünstig herstellen zu können, sind mehrnutzige bzw. mehrspurige Verpackungsanlagen erforderlich. Die Figuren 1 bis 3 zeigen Bereiche von Bearbeitungsstationen, auf denen einige Verfahrensschritte zur Pflasterverpackung stattfinden.

Eine erste Station ist die Synchronisationsstation (30), vgl. Figur 1. Diese Station umfasst ein z.B. auf zwei Rollen (35, 36) geführtes und angetriebenes Transportelement (33). Letzteres ist hier ein endloses Transportband, beispielsweise eine dünnwandige fluorsilikoniert beschichtete Metallfolie. In Längsrichtung (19) gesehen befinden sich hinter dem Transportband (33) eine nicht angetriebene Achse (21), auf der zwei Folienrollen (23, 24) gelagert sind. Jede Folienrolle (23, 24) besteht aus einer sehr langen, schmalen aufgewickelten Reservoirfolienbahn (15, 16). Da die Reservoirfolienbahnen (15, 16) auch auf einspurigen Anlagen verpackt bzw. konfektioniert werden, haben die Rollen (15, 16) bei einem Chargenneustart auf einer mehrspurigen Anlage meist unterschiedliche Durchmesser.

Jeder Reservoirfolienbahn (15, 16) wird dem Transportband (33) mit der klebenden Haftschicht (3) nach unten zugeführt und mittels der ersten Andruckrolle (31) dort angelegt. Bei der Anlage entsteht eine temporär haftende Verbindung mit dem Transportband (33). In der Folge werden die Reservoirfolienbahnen (15, 16) nur durch die ziehende Bewegung des Transportbandes (33) synchron parallel zueinander befördert. Auf dem Transportband (33) haben die beiden Reservoirfolienbahnen (15, 16) z.B. einen Abstand, der dem doppelten Randbereich (11) der Schutzfolie (5) entspricht. Das Transportband (33) ist breiter als die Summe der nebeneinander aufliegenden Reservoirfolienbahnen (15, 16) einschließlich der Bahnzwischenräume. Anstelle des endlosen Transportbandes (33) kann auch eine zylindrische Walze verwendet werden.

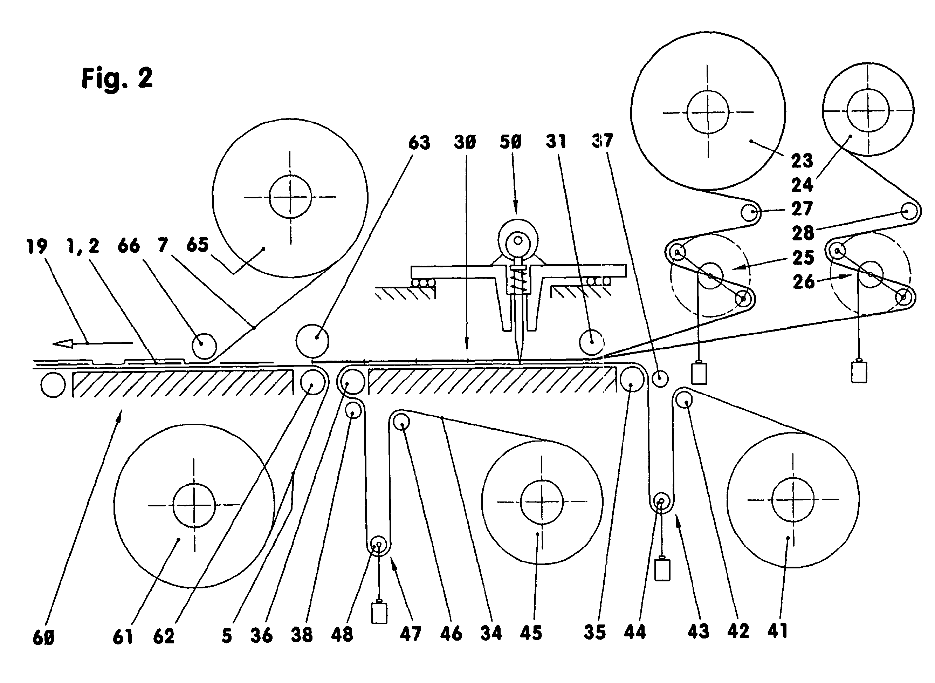

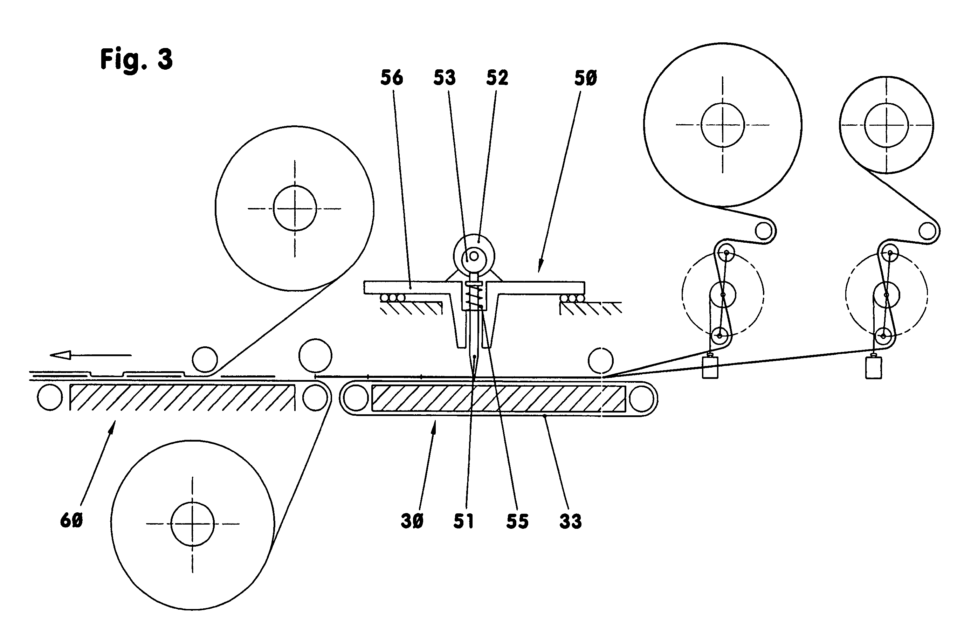

Die Pendelpuffer (25, 26) aus den Figuren 2 und 3 sind aus Vereinfachungsgründen in Figur 1 nicht dargestellt.

In Figur 2 wird ein Transportelement (34) dargestellt, das anstelle des endlosen Transportbandes (33) eine einmal verwendbare Kunststofffolie vorsieht. Diese Hilfsfolie (34), z.B. eine silikonierte Polyesterfolie, wird von einer angetriebenen Spenderolle (41) abgewickelt, durchläuft eine erste Pufferschleife (43), umschlingt eine erste und zweite Umlenkrolle (35, 36), passiert eine zweite Pufferschleife (47) und wird schließlich auf einer angetriebenen Sammelrolle (45) wieder aufgewickelt. Die zweite Umlenkrolle (36) erzeugt den Transportvorschub. Eine Andruckrolle (38) verlängert durch ihre Lage unterhalb der Umlenkrolle (36) den Umschlingungsbogen der Kunststofffolie (34). Die Pufferschleifen (43, 47), die durch ihre Auf- und Abwärtsbewegungen die diskontinuierliche Hilfsfolienbewegung ausgleichen und so die Antriebe der Rollen (41, 45) vor Beschleunigungsänderungen schützen, werden z.B. durch gewichtsbelastete Spannrollen (44. 48) gespannt.

Oberhalb der Synchronisationsstation (30) ist eine nur schematisch dargestellte Trennvorrichtung (50) angeordnet, vgl. Figur 3. Letztere trennt mit einem hier z.B. quer zur Längsrichtung (19) ausgerichteten Trennwerkzeug, beispielsweise einem Schneidmesser (51) die jeweiligen Reservoirfolien (1, 2) von den entsprechenden Reservoirfolienbahnen (15, 16) ab. Für die Trennbewegung des Scheidmessers (51) wirkt ein Exzenter- bzw. Trennantrieb (52, 53) auf das Messer (51). Den Rückhub des Messers (51) bewirkt ein Federelement (55). Das Schneidmesser (51) und der Antrieb (52, 53) sind Teil eines Schlittens (56), der nach dem Ausführungsbeispiel während des Scheidvorganges zumindest bereichsweise synchron zum Transportelement (33, 34) bewegt wird.

Selbstverständlich kann die Trennbewegung auch in der Vorschubpause des Transportelements (33, 34) stattfinden. In diesem Fall verharrt der Schlitten (56) - ohne gleit- oder wälzgelagerte Längsführung - ortsfest gegenüber der Synchronisationsstation (30).

Werden für den Trennvorgang mechanische Stanz- oder Schneidwerkzeuge eingesetzt, so kann zwischen dem Trennantrieb (52, 53) und dem Trennwerkzeug (51) ein Ausgleichspufferelement angeordnet werden, um beim Trennvorgang das entsprechende Transportelement (33, 34) nicht mechanisch zu beschädigen. Bei optischen oder hydraulischen Trennverfahren entfällt ein derartiges Element.

In den Figuren 2 und 3 sind zwischen den hier versetzt zueinander dargestellten Folienrollen (23), (24) und der oberen Abdruckrolle (31) jeweils ein gewichtsbelasteter Pendelpuffer (25, 26) mit jeweils vorgeschalteter Umlenkrolle (27, 28) angeordnet. Durch den Einbau der Pendelpuffer (25, 26) drehen sich die z.B. nicht angetriebenen, trägen Folienrollen (23, 24) bei den Abbremsvorgängen des Transportelements (33, 34) weiter, so dass die relativ dünnen Reservoirfolienbahnen (15, 16) keinen unnötigen Zugspannungsschwankungen ausgesetzt werden.

Der Synchronisationsstation (30) nachgeschaltet ist eine Verpackungsstation (60). In dieser wird die Schutzfolie (5) von einer z.B. nicht angetriebenen Schutzfolienrolle (61) abgewickelt und nach einer Umlenkung durch die Transportrolle (62) mit Hilfe einer nicht dargestellten einen Vorschub erzeugenden Einrichtung in Längsrichtung (19) gezogen. Auf die sich beispielsweise kontinuierlich bewegende Schutzfolie (5) werden die haftklebenden Reservoirfolien (1, 2) - im Ausführungsbeispiel paarweise - übergeben. Dort werden sie über mindestens eine Anpresswalze (63) auf die Schutzfolie (5) aufgewalzt.

Durch die Abstimmung der Bewegungen des Transportelements (33, 34) und der Schutzfolie (5) wird zwischen den vereinzelten Reservoirfolien (1, 2) ein konstanter Abstand erzeugt.

Hinter der Anpresswalze (63) wird die Rückfolie (7) an den neuen Verbund aus Schutzfolie (5) und aufliegenden Reservoirfolien (1, 2) herangeführt. Die von einer z.B. nicht angetriebenen Rückfolienrolle (65) abgewickelte Rückfolie (7) wird mit ihrer nach unten orientierten Klebeschicht (6) u.a. mittels der Anpresswalze (66) auf den Verbund (1, 2; 5) dicht aufgeklebt.

In weiteren Stationen werden die Ränder (8) der Rückfolie (7) geschnitten und die hierbei entstehenden gitterförmigen Rückfolienreste abgezogen. In einem weiteren Schritt werden die Pflaster durch Schneiden der Schutzfolienränder (12) vereinzelt.

Im Ausführungsbeispiel wird die Mehrnutzigkeit bzw. die Mehrspurigkeit des Verfahrens an einer zweispurigen Anlage gezeigt. Je nach Pflasterbreite können derartige Anlagen problemlos auch auf z.B. 10 oder noch mehr Spuren umgerüstet werden.

### Bezugszeichenliste:

- 1, 2: Reservoirfolie, Wirkstoffreservoir
- 3: Haftklebeschicht von (1, 2)
- 4: Sperrschicht von (1, 2)
- 5: Schutzfolie, abziehbar
- 6: Rückfolie
- 7: Klebeschicht von (6)
- 8: Rand von (6)
- 9: Stanzlinie von (5)

- 11: Randbereich
- 12: Schutzfolienrand
- 13, 14: Konfektionierungsspuren
- 15, 16: Reservoirfolienbahn
- 17: Reservoirfolienlänge
- 19: Längsrichtung, Transportrichtung

- 21: Achse
- 23: Rolle, Folienrolle, groß
- 24: Rolle, Folienrolle, klein
- 25, 26: Pendelpuffer
- 27, 28: Umlenkrollen

- 30: Synchronisationsstation
- 31: Andruckrolle, erste, oben
- 33: Transportband, Transportelement
- 34: Hilfsfolie, Transportelement
- 35: Umlenkrolle, erste
- 36: Umlenkrolle, zweite, angetrieben
- 37, 38: Andruckrollen, unten

- 41: Spenderolle
- 42: Umlenkrolle
- 43: Pufferschleife, erste
- 44: Spannrolle

- 45: Sammelrolle
- 46: Umlenkrolle
- 47: Pufferschleife, zweite
- 48: Spannrolle

- 50: Trennvorrichtung
- 51: Schneidmesser, Messer, Trennwerkzeug
- 52: Motor, Teil des Trennantriebs
- 53: Exzenter, Teil des Trennantriebs
- 55: Federelement, Rückholfeder
- 56: Schlitten

- 60: Verpackungsstation
- 61: Schutzfolienrolle
- 62: Transportrolle
- 63: Anpresswalze

- 65: Rückfolienrolle
- 66: Anpresswalze

## Patentansprüche

1. Verfahren zum mehrspurigen Konfektionieren transdermaler therapeutischer Pflaster mit jeweils einer mindestens einlagigen, wirkstoffhaltigen und haftklebenden Reservoirfolie (1, 2), die zwischen einer Rückfolie (30) und einer ablösbaren Schutzfolie (20) angeordnet wird,
- wobei pro Konfektionierungsspur (13, 14) die Reservoirfolie (1, 2) jeweils als Reservoirfolienbahn (15, 16) auf einer Rolle (23, 24) aufgewickelt vorliegt,
- wobei die Reservoirfolienbahnen (1, 2) pro Spur (13, 14) mit ihrer Haftklebeschicht (3) auf ein diskontinuierlich in Längsrichtung (19) der Reservoirfolienbahnen (15, 16) bewegtes Transportelement (33, 34) aufgelegt werden,
- wobei alle auf dem Transportelement (33, 34) nebeneinander aufliegenden Reservoirfolienbahnen (15, 16) quer oder schräg zur Längsrichtung (19) zumindest annähernd zeitgleich in einzelnen Reservoirfolien (1, 2) mit Hilfe einer Trennvorrichtung (50) abgetrennt werden,
- wobei die jeweils nebeneinander liegenden Reservoirfolien (1, 2) am Transportelementende an ein kontinuierlich bewegtes weiteres Transportelement (5) zur beabstandenden Ablage übergeben werden,
- wobei nach der Übergabe der Reservoirfolien (1, 2) das Transportelement (33, 34) kurz abgebremst wird.

2. Verfahren gemäß Anspruch 1, **dadurch gekennzeichnet, dass** die Konfektionierungsspuren (13, 14) parallel zueinander verlaufen.

3. Verfahren gemäß Anspruch 1, **dadurch gekennzeichnet, dass** die einzelnen Reservoirfolienbahnen (15, 16) der entsprechenden Konfektionierungsspuren (13, 14) gemeinsam mit einer Andruckrolle (31) auf das Transportelement (33, 34) gepresst werden.

4. Verfahren gemäß Anspruch 3, **dadurch gekennzeichnet, dass** die einzelnen Reservoirfolienbahnen (15, 16) zwischen den Folienrollen (23, 24) und der Andruckrolle (31) mit Hilfe von Folienpuffer (25, 26) mehrfach umgelenkt werden.

5. Verfahren gemäß Anspruch 1, **dadurch gekennzeichnet, dass** beide Transportelemente (33, 34; 5) in der gemeinsamen Bewegungsphase die gleiche Vorschubgeschwindigkeit haben.

6. Verfahren gemäß Anspruch 1, **dadurch gekennzeichnet, dass** die Trennvorrichtung (50) auf einem wälz- oder gleitgelagerten Schlitten (56) angeordnet ist und mit diesem (56) parallel und bereichsweise synchron zum Transportelement (33, 34) mitbewegt wird.

7. Verfahren gemäß Anspruch 6, **dadurch gekennzeichnet, dass** nach der Übergabe der Reservoirfolien (1, 2) an das Transportelement (5) die Trennvorrichtung (50) bei stillstehendem Transportelement (33, 34) eine mittlere Reservoirfolienlänge (17) zurückfährt, um mit einem neuen Trennvorgang und einer neuen Vorschubbewegung des Transportelementes (33, 34) zu beginnen.

## Claims

1. A method for multi-track production of transdermal therapeutic patches with in each case a pressure-sensitive adhesive reservoir film (1, 2) which comprises at least one layer and contains active substance and which is arranged between a backing film (30) and a removable protective film (20),
- wherein the reservoir film (1, 2) for each production track (13, 14) is in each case wound as a reservoir film web (15, 16) on a roller (23, 24),
- wherein the reservoir film webs (1, 2) for each track (13, 14) are placed with their pressure-sensitive adhesive layer (3) onto a conveyor element (33, 34) that is moved non-continuously in the longitudinal direction (19) of the reservoir film webs (15, 16),
- wherein all the reservoir film webs (15, 16) lying next to one another on the conveyor element (33, 34) are separated off into individual reservoir films (1, 2) with the aid of a separating device (50), transversely or obliquely with respect to the longitudinal direction (19) and at least approximately simultaneously,
- wherein the reservoir films (1, 2) each lying next to one another at the end of the conveyor element are transferred to a continuously moved additional conveyor element (5) and placed at intervals thereon,
- wherein the conveyor element (33, 34) is briefly slowed down after the transfer of the reservoir films (1, 2).

2. The method as claimed in claim 1, **characterized in that** the production tracks (13, 14) extend parallel to one another.

3. The method as claimed in claim 1, **characterized in that** the individual reservoir film webs (15, 16) of the corresponding production tracks (13, 14) are pressed jointly onto the conveyor element (33, 34) by a pressure roller (31).

4. The method as claimed in claim 3, **characterized in that** the individual reservoir film webs (15, 16) are deflected several times between the film rollers (23, 24) and the pressure roller (31) with the aid of film buffers (25, 26).

5. The method as claimed in claim 1, **characterized in that** both conveyor elements (33, 34; 5) have the same speed of advance in the common phase of movement.

6. The method as claimed in claim 1, **characterized in that** the separating device (50) is arranged on a carriage (56) mounted on rollers or slides and is moved together with the latter (56) in parallel and in some areas in synchrony with the conveyor element (33, 34).

7. The method as claimed in claim 6, **characterized in that**, after the transfer of the reservoir films (1, 2) to the conveyor element (5), the separating device (50) travels back an average reservoir film length (17), with the conveyor element (33, 34) stationary, in order to begin with a new separating operation and a new advance movement of the conveyor element (33, 34).

## Revendications

1. Procédé pour confectionner plusieurs bandes de pansements transdermiques avec chacune un film réservoir (1, 2) autoadhésif, en au moins une couche et contenant une substance active, qui est disposé entre un film dorsal (30) et un film de protection amovible (20),
- dans lequel, pour chaque ligne de confection (13, 14), le film réservoir (1, 2) se présente chaque fois sous la forme d'une bande de film réservoir (15, 16) enroulée sur une bobine (23, 24),
- dans lequel les bandes de film réservoir (1, 2) sont déposées par ligne (13, 14) avec leur couche adhésive (3) sur un élément de transport (33, 34) déplacé de façon discontinue dans la direction longitudinale (19) des bandes de film réservoir (15, 16),
- dans lequel toutes les bandes de film réservoir (15, 16) reposant l'une à côté de l'autre sur l'élément de transport (33, 34) sont coupées, transversalement ou en oblique par rapport à la direction longitudinale (19), au moins à peu près simultanément en films réservoirs individuels (1, 2) à l'aide d'un dispositif de coupe (50),
- dans lequel les films réservoirs (1, 2) reposant chaque fois l'un à côté de l'autre sont transférés, à l'extrémité de l'élément de transport, à un autre élément de transport déplacé en continu (5) en vue d'un dépôt espacé,
- dans lequel l'élément de transport (33, 34) est brièvement freiné après le transfert des films réservoirs (1, 2).

2. Procédé selon la revendication 1, **caractérisé en ce que** les lignes de confection (13, 14) s'étendent parallèlement l'une à l'autre.

3. Procédé selon la revendication 1, **caractérisé en ce que** les bandes individuelles de film réservoir (15, 16) des lignes de confection correspondantes (13, 14) sont pressées ensemble sur l'élément de transport (33, 34) avec un rouleau de pression (31).

4. Procédé selon la revendication 3, **caractérisé en ce que** les bandes individuelles de film réservoir (15, 16) sont déviées plusieurs fois entre les bobines de film (23, 24) et le rouleau de pression (31) à l'aide d'un accumulateur de film (25, 26).

5. Procédé selon la revendication 1, **caractérisé en ce que** les deux éléments de transport (33, 34; 5) présentent la même vitesse d'avance pendant la phase de déplacement commune.

6. Procédé selon la revendication 1, **caractérisé en ce que** le dispositif de coupe (50) est disposé sur un chariot à roulement ou à glissement (56) et est entraîné avec celui-ci (56) parallèlement et localement en synchronisme avec l'élément de transport (33, 34).

7. Procédé selon la revendication 6, **caractérisé en ce que**, après le transfert des films réservoirs (1, 2) à l'élément de transport (5), le dispositif de coupe (50) exécute un mouvement de retour d'une longueur moyenne de film réservoir (17) pendant que l'élément de transport (33, 34) est à l'arrêt, afin de commencer une nouvelle opération de coupe et un nouveau déplacement d'avance de l'élément de transport (33, 34).
